# FASCICULE DE BREVET EUROPEEN

(11) **EP 0 915 858 B1**
(45) Date de publication et mention de la délivrance du brevet: **13.09.2000**
(21) Numéro de dépôt: 97934587.3
(22) Date de dépôt: 17.07.1997
(51) Int. Cl.: C07D 277/36, C07D 417/04, C07D 233/86

(54) **INTERMEDIAIRES POUR LA PREPARATION DE 2-IMIDAZOLINE-5-ONES**
Zwischenprodukte um 2-imidazolin-5onederivate herzustellen.
INTERMEDIATES FOR THE PREPARATION OF 2-IMIDAZOLINE-5-ONES

(30) Priorité: 22.07.1996 FR 9609483
(43) Date de publication de la demande: 19.05.1999
(73) Titulaire: AVENTIS CROPSCIENCE S.A., 69009 Lyon (FR)
(72) Inventeur: BUFORN, Albert, F-69008 Lyon (FR); GADRAS, Alain, F-69009 Lyon (FR)
(86) Numéro de dépôt international: FR9701334
(87) Numéro de publication internationale: WO9803490

(56) Documents cités:
- GB-A- 2 035 998
- ERNST SCHAUMANN ET AL: "Konkurrenz zwischen 1,2- und 1,3-Ringöffnung in der Umsetzung von 3-Dimethylamino-2-phenyl-2H-azirinen mit Kohlenstoffdisulfid" TETRAHEDRON LETTERS, no. 16, avril 1977, OXFORD GB, pages 1351-1354, XP002027556

## Description

La présente invention concerne de nouveaux produits utilisables à titre d'intermédiaires pour la préparation de 2-imidazoline-5-ones à usage fongicide. Elle concerne également les procédés de préparation de ces nouveaux produits ainsi qu'un procédé utile pour l'obtention de ces 2-imidazoline-5-ones à partir de ces nouveaux intermédiaires.

On connaît, notamment par les demandes de brevet européen EP 551048, EP 599749. EP 629616 et par la demande internationale WO 93/24467 des 2-imidazoline-5-ones à usage fongicide.

Un but de la présente invention est de proposer de nouveaux intermédiaires permettant la préparation de ces 2-imidazoline-5-ones.

Un autre but de la présente invention est de proposer une nouvelle voie d'accès aux 2-imidazoline-5-ones fongicides présentant une sécurité améliorée.

L'invention a ainsi pour objet, en premier lieu, des 2-thio-thiazolidine-5-ones de formule générale (I): 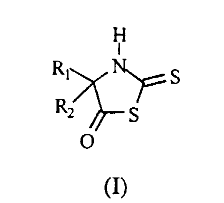 dans laquelle :
- R¹ est un radical C₁-C₃ alkyle ou phényle.
- R² est un groupe aryle choisi parmi phényle ou pyridyle éventuellement substitué par 1 à 3 groupes choisis parmi un atome d'halogène, un groupe nitro, cyano, un radical C₁-C₃ alkyl, C₁-C₃ alkoxy ;
à l'exception de la 4-éthyl-4-phényl-2-thio-thiazolidine-5-one décrite dans Tetrahedron Letters, 16, (1977), 1351-1354.

L'invention a également pour objet les formes salifiées, ainsi que les stéréoisomères des composés de formule (I). Elle a notamment pour objet les isomères optiques résultant de la présence d'un carbone asymétrique, et tout particulièrement, lorsque les radicaux R¹ et R² sont différents, les isomères optiques découlant de la présence du carbone asymétrique porteur de R¹ et R². Ces isomères optiques sont des composés optiquement purs ou fortement enrichis en un énantiomère. Dans ce qui suit, on entend par composé optiquement actif fortement enrichi en un énantiomère déterminé, un composé contenant au moins 80%, de préférence au moins 90% de cet énantiomère. Tous ces composés sont considérés comme compris dans la formule (I) précédemment définie.
Parmi les composés de formule (I), on préfère ceux pour lesquels :
- R¹ représente un radical C₁-C₃ alkyle,
- R² représente un phényle éventuellement substitué par un atome d'halogène, un groupe cyano, nitro, un radical méthyle ou méthoxy.

On préfère encore plus particulièrement parmi les composés de formule (I) ceux pour lesquels R² est un phényle, et R¹ est un méthyle.

Selon une variante très avantageuse de l'invention, le composé de formule (I) dans laquelle :
- R¹ est un méthyle, et
- R² est un phényle,
est un énantiomère relativement au carbone asymétrique porteur de R¹ et R².

Dans la présente description, tous les groupes apparaissant dans les formules chimiques qui suivent, et qui ont déjà été définis dans la formule générale (I), conservent la même signification, pour autant qu'il n'en soit pas précisé autrement. Les radicaux alkyle mentionnés dans le présent texte peuvent être linéaires ou ramifiés.

On décrit à présent un mode de préparation du composé de formule (I). Ce mode de préparation est indiqué dans le cas de composés qui sont racémiques relativement au carbone porteur des radicaux R¹ et R². L'homme du métier peut cependant utiliser ces mêmes réactions lorsqu'il souhaite obtenir un composé de formule (I) énantiomère relativement au carbone porteur de R¹ et R². En effet, les réactions indiquées ci-après sont parfaitement stéréosélectives, dans le sens où elles n'entraînent pas de modification de la configuration absolue de ce même carbone.

Le composé de formule I peut être obtenu en faisant réagir un composé de formule II avec du sulfure de carbone, dans un solvant ou un mélange de solvants, éventuellement en présence d'une base, à une température comprise entre 0°C et + 50°C selon le schéma suivant : 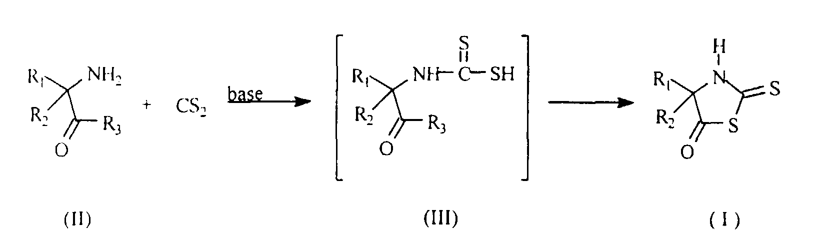 dans lequel R³ est un groupe amino, hydroxy, ou un radical alkoxy, linéaire ou ramifié, contenant de 1 à 6 atomes de carbone, de préférence de 1 à 3, ou un radical benzyloxy éventuellement substitué par un atome d'halogène.

La base éventuellement utilisée peut être une base minérale telle qu'un hydroxyde ou un carbonate de métal alcalin ou alcalino-terreux ou une base organique telle qu'une amine primaire, secondaire ou tertiaire. Elle peut être utilisée dans un rapport (exprimé en nombre de moles) base/ composé II compris entre 0,05 et 1,2 , de préférence compris entre 0,1 et 1.

Dans ce schéma, le composé de formule (III) peut être isolé à titre d'intermédiaire dans le cas où une base est utilisée, sous la forme d'un sel.

Comme solvant on peut utiliserl'eau, les éthers, les éthers cycliques, les esters d'alkyles, les solvants dipolaires tel l'acétonitrile, les alcools de 1 à 4 atomes de carbone, les solvants aromatiques, de préférence le toluène, le dichlorométhane ou le chloroforme, le sulfure de carbone. Comme mélange de solvants, on peut utiliser le mélange d'un ou plusieurs alcools avec l'un ou plusieurs des solvants précités.

Lorsque R³ est le groupe hydroxy, on préfère utiliser l'eau comme solvant.

Lorsque R³ est autre que le groupe hydroxy, on préfère utiliser un mélange alcool/eau comme solvant.

Dans le cas où le composé de formule (III) est isolé, il peut être transformé directement en composé (I) par chauffage à une température allant de 25°C au reflux du solvant utilisé. La transformation du composé intermédiaire (III) en composé (I) peut également être réalisée par traitement avec un acide fort qui est soit un acide minéral tel que l'acide chlorhydrique ou sulfurique, soit un acide organique tel que l'acide trifluoroacétique.

On préfère mettre en oeuvre ce procédé en l'absence de base ou à une température comprise entre 20 et 40 °C. Dans ce cas, le composé de formule (III) n'est pas isolé.

D'autres modalités de la procédure permettant de préparer le composé (I) au départ de (II) sont décrites par A.C. DAVIS et A.J LEVY dans J. Chem. Soc., pp 2419-25 (1951) ou par K. HOFMANN et coll. dans J.Am. Chem. Soc., vol 74, pp 470-476(1952).

L'α-amino-ester de formule (II) dans laquelle R₃ est un radical alkoxy peut être obtenu par estérification des α-aminoacides correspondants selon un procédé analogue à celui décrit par M. Brenner et W. Huber dans Helv Ch. Acta.(1953) Volume :36 pages:1109 - 1115.

L'α-amino amide de formule (II) dans laquelle R₃ est un groupe amino peut être obtenu à partir d'un aminoester par action de l'ammoniac comme décrit par J. A. Garbarino dans Ann. Chimica Ital. vol. 59, pp 841-849 (1969).

Les α-aminoacides sont préparées par des réactions et méthodes connues en soi.

Lorsque le composé de formule (II) est un énantiomère d'amino-ester, il peut être obtenu notamment par :
- amination diastéréosélective d'un composé prochiral suivie d'une déprotection de la copule chirale comme décrit par R.S.ATKINSON et coll., Tetrahedron, 1992, 48, pp 7713-30, ou bien par
- dédoublement du racémique correspondant avec un composé chiral comme décrit par Y.SUGI et S.MITSUI, Bull. Chem. Soc. Japan, 1969, 42, pp 2984-89, ou bien par
- estérification d'un aminoacide chiral comme décrit par D.J.CRAM et coll., J.Am.Chem.Soc., 1961, 83, pp 2183-89.

Lorsque le composé de formule (II) est un énantiomère d'amino-amide, il peut être obtenu soit au départ d'un amino-ester chiral, soit par dédoublement du racémique correspondant comme exposé par H. DAHN et coll. dans Helv. Chim. Acta, vol. 53, pp 1370-1378 (1970).

Les 2-thio-thiazolidine-5-ones de formule (I) sont utiles pour la préparation de 2-imidazoline-5-ones fongicides de formule (IV) : 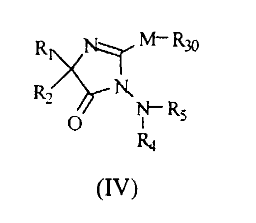 dans laquelle :
- M représente l'atome d'oxygène ou de soufre
- R³⁰ représente un radical alkyle, linéaire ou ramifié, contenant de 1 à 6 atomes de carbone, ou un radical haloalkyle, linéaire ou ramifié, contenant de 1 à 6 atomes de carbone;
- R⁴ représente un atome d'hydrogène ou un radical acyle ;
- R⁵ représente un radical aryle ou hétéroaryle choisi parmi : phényle, naphtyle, pyridinyle, pyrimidinyle, pyridazinyle, pyrazinyle, thiényle, benzothiényle, furyle benzofuryle, quinolinyle, isoquinolinyle, ou méthylène dioxyphényle, chacun de ces radicaux étant éventuellement substitué par 1 à 7 groupements, identiques ou différents, de préférence de 1 à 3, choisis parmi les significations de R⁵¹ défini ci-après ;
- R⁵¹ représente :
   - un atome d'halogène, ou
   - un radical alkyle, haloalkyle, alkoxy, haloalkoxy, alkylthio, haloalkylthio ou alkylsulfonyl, linéaire ou ramifié, de 1 à 6 atomes de carbone, ou
   - un radical cycloalkyle, halocycloalkyle, alcényloxy, alcynyloxy, alcénylthio, alcynylthio de 3 à 6 atomes de carbone, ou
   - le groupe nitro ou cyano, ou
   - un radical amino éventuellement mono ou disubstitué par un radical alkyl ou acyl de 1 à 6 atomes de carbone ou alkoxycarbonyl de 2 à 6 atomes de carbone ;
ainsi que les formes salifiées acceptables en agriculture de ces composés et leurs stéréoisomères, notamment, lorsque R¹ et R² sont différents, les isomères optiques découlant de la présence du carbone asymétrique porteur des radicaux R¹ et R².

On décrit à présent la préparation des composés fongicides de formule (IV) à partir des 2-thio-thiazolidine-5-ones de formule (I) objets de l'invention, selon un procédé utilisable aussi bien en série racémique qu'en série énantiomèrique.

On fait réagir le composé de formule (I) sur un composé de formule (V) dans un solvant, et à une température comprise entre +20°C et +100°C, de préférence entre 40 et 80 °C, selon le schéma suivant :

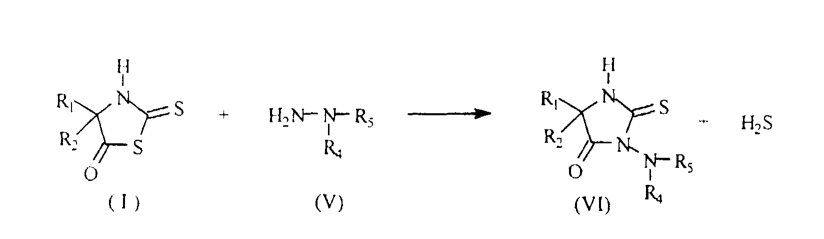

Comme solvant, on peut utiliser un éther par exemple le dioxane, un solvant aprotique dipolaire, notamment la N-méthylpyrolidone, le diméthylformamide, le dimethylsulfoxyde ou l'acétonitrile, un alcool comprenant de 1 à 4 atomes de carbone et plus particulièrement le méthanol, un solvant aromatique et plus précisément la pyridine ou le monochlorobenzène.

On préfère mettre en oeuvre cette réaction en utilisant un catalyseur choisi parmi une amine tertiaire telle que la triéthylamine ou la tributylamine, ou un sel organique de cette amine, tel que l'acétate de tributylamine. Ce catalyseur est présent dans une proportion catalyseur/composé (I) (exprimée en nombre de moles) allant de 0,05 à 1 , de préférence de 0,1 à 0,5. On obtient dans ce cas une pureté améliorée.

La thiohydantoïne de formule (VI) est transformée en 2-imidazoline-5-one de formule (IV) selon un procédé décrit dans l'une des demandes de brevet EP 551048, EP 599749 ou EP 629616.

Les exemples qui suivent sont donnés à titre purement illustratif des composés et procédés de préparation objets de l'invention. Ils ne sont en aucun cas limitatifs de celle-ci. La structure du dérivé illustré a été établie à l'aide d'au moins une des techniques spectrales suivantes : sptectrométrie RMN du proton, spectrométrie RMN du carbone 13, spectrométrie Infra-Rouge et spectrométrie de masse, ainsi que les méthodes usuelles de mesure des pouvoirs rotatoires.

### Exemple N°1 : Préparation de la (4-S) 4-méthyl-4-phényl-2 thio-thiazolidine-5-one à partir d'un amino-amide :

Dans un ballon de 50 ml muni d'une agitation mécanique, on introduit 3,26 g (20 mmoles) de (2-S) 2-amino-2-phényl propionamide, 6 ml (100 mmoles) de sulfure de carbone et 4 ml d'acétonitrile. Le milieu hétérogène est maintenu 20 h sous agitation à 20°C. Après distillation sous vide du sulfure de carbone en excès et de l'acétonitrile, on obtient après purification et filtration 3,70g de (4-S) 4-méthyl-4-phényl-2 thio-thiazolidine-5-one sous la forme d'une solide blanc fondant à 104°C , correspondant à un rendement de 83%.

### Exemple N°2 : Préparation de la (4-S) 4-méthyl-4-phényl-2 thio-thiazolidine-5-one à partir d'un amino-ester :

Dans un tube à essai de 25 ml, muni d'une agitation magnétique, on introduit 2 g (11,1 mmole) de (2-S) 2-amino-2-phényl propionate de méthyle, 15 ml de tetrahydrofurane, 1,82 ml (13 mmole) de triéthylamine et 0,78 ml (13 mmole) de sulfure de carbone. Après fermeture hermétique, le tube à essai est maintenu à 45 °C et le milieu homogène est maintenu 4 h sous agitation à cette température.
Après refroidissement et purification, on obtient 2 g de (4-S) 4-méthyl-4-phényl-2 thio-thiazolidine-5-one sous la forme d'une poudre blanche soit un rendement de 80%.

### Exemple N°3 : Préparation de la (4-S) 4-méthyl 4-phényl-1-phénylamino-2-thiohydantoine :

Dans un ballon de 25 ml, muni d'une agitation magnétique, on introduit 893 mg (4 mmole de (4-S) 4-méthyl-4-phényl-2-thio-thiazolidine-5-one, 8 ml d'acétonitrile et 100 µl (0,4 mmole) de tributylamine. Le mélange est chauffé à 70°C puis on coule une solution de 520 mg (4,8 mmole) de phénylhydrazine dans 4,5 ml d'acétonitrile pendant 2 h. Le milieu réactionnel est chauffé à 80°C pendant 6 h. Après refroidissement, l'acétonitrile est éliminé par distillation sous pression réduite. Après purification, on obtient 975 mg de (4-S) 4-méthyl-4-phényl-1-phénylamino-2-thiohydantoine sous la forme d'un solide blanc fondant à 167°C dont la pureté mesurée par HPLC est de 100 % soit un rendement de 82 %.

### Exemple n° 4 : Préparation de la (4-S) 4-méthyl-4-phényl-2 thio-thiazolidine-5-one à partir d'acide (2S)-2-amino-2-phényl propionique :

Dans un ballon de 50 ml, muni d'une agitation magnétique, on introduit successivement 11 g (10⁻² mole ) d'acide (2S)-2-amino-2-phényl propionique (sous forme d'un mélange à 15 % en poids, d'amino acide dans NaCl solide), 10 ml de N-methylpyrrolidone, 0,8 g (2.10⁻² mole) de NaOH en pastille, puis 1,8 ml (3. 10⁻²) de disulfure de carbone. Après fermeture hermétique, le milieu réactionnel est chauffé à 60°C pendant 5 heures, sous vive agitation.

Après refroidissement, 50 ml d'eau puis 5,4 ml d' H₂SO₄ concentré sont ajoutés au milieu réactionnel. On procède à l'extraction de la phase organique, qui est lavée, séchée puis concentrée. On obtient la (4-S) 4-méthyl-4-phényl-2 thio-thiazolidine-5-one avec un rendement de 84 %.

## Revendications

1. Composé 2-thio-thiazolidine-5-one de formule générale (I): 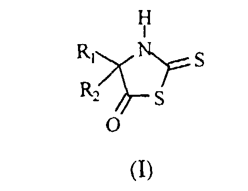 dans laquelle :
- R¹ est un radical C₁-C₃ alkyle ou phényle.
- R² est un groupe aryle choisi parmi phényle ou pyridyle éventuellement substitué par 1 à 3 groupes choisis parmi un atome d'halogène, un groupe nitro, cyano, un radical C₁-C₃ alkyl, C₁-C₃ alkoxy ;
ainsi que les formes salifiées, et les stéréoisomères correspondants et notamment, lorsque les radicaux R¹ et R² sont différents, les isomères optiques découlant de la présence du carbone asymétrique porteur de R¹ et R² ;
à l'exception de la 4-éthyl-4-phényl-2-thio-thiazolidine-5-one .

2. Composé selon la revendication 1, caractérisé en ce que :
- R¹ représente un radical C₁-C₃ alkyle,
- R² représente un phényle éventuellement substitué par un atome d'halogène, un groupe cyano, nitro, un radical méthyle ou méthoxy.

3. Composé selon l'une des revendications 1 ou 2, caractérisé en ce que R² est un phényle, et R¹ est un méthyle.

4. Composé selon l'une des revendications 1 à 3, caractérisé en ce qu'il est un énantiomère relativement au carbone asymétrique porteur de R¹ et R².

5. Procédé de préparation du composé de formule (I), tel que défini dans l'une des revendications 1 à 4, caractérisé en ce que l'on fait réagir un composé de formule II avec du sulfure de carbone, dans un solvant ou un mélange de solvants, éventuellement en présence d'une base, à une température comprise entre 0°C et + 50°C selon le schéma : 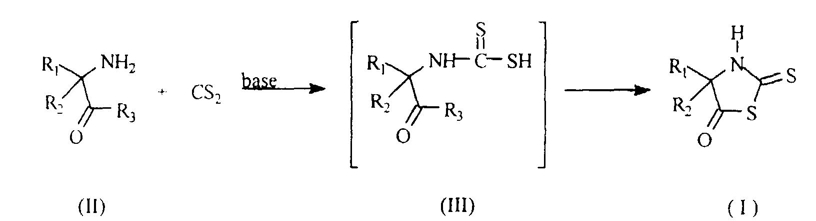 dans lequel R³ est un groupe amino, hydroxy ou un radical alkoxy, linéaire ou ramifié, contenant de 1 à 6 atomes de carbone, de préférence de 1 à 3, ou un radical benzyloxy éventuellement substitué par un atome d'halogène.

6. Procédé de préparation selon la revendication 5, caractérisé en ce qu'il est mis en oeuvre en l'absence de base ou à une température comprise entre 20 et 40 °C.

7. Procédé de transformation du composé de formule (I) tel que défini dans l'une des revendications 1 à 4, caractérisé en ce qu'on le fait réagir sur un composé de formule (V) dans un solvant, et à une température comprise entre +20°C et +100°C, de préférence entre 40 et 80 °C, selon le schéma : 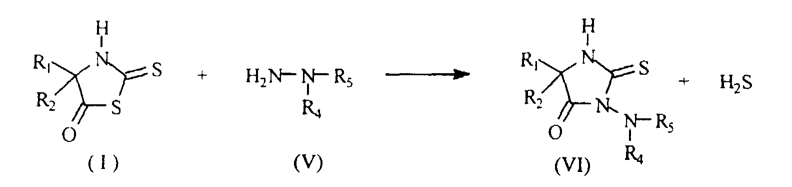 dans lequel :
- R⁴ représente un atome d'hydrogène ou un radical acyle ;
- R⁵ représente un radical aryle ou hétéroaryle choisi parmi : phényle, naphtyle, pyridinyle, pyrimidinyle, pyridazinyle, pyrazinyle, thiényle, benzothiényle, furyle benzofuryle, quinolinyle, isoquinolinyle, ou méthylène dioxyphényle, chacun de ces radicaux étant éventuellement substitué par 1 à 7 groupements, identiques ou différents, de préférence de 1 à 3, choisis parmi les significations de R⁵¹ ;
- R⁵¹ représente :
- un atome d'halogène, ou
- un radical alkyle, haloalkyle, alkoxy, haloalkoxy, alkylthio, haloalkylthio ou alkylsulfonyl, linéaire ou ramifié, de 1 à 6 atomes de carbone, ou
- un radical cycloalkyle, halocycloalkyle, alcényloxy, alcynyloxy, alcénylthio, alcynylthio de 3 à 6 atomes de carbone, ou
- le groupe nitro ou cyano, ou
- un radical amino éventuellement mono ou disubstitué par un radical alkyl ou acyl de 1 à 6 atomes de carbone ou alkoxycarbonyl de 2 à 6 atomes de carbone.

8. Procédé de transformation selon la revendication 7, caractérisé en ce qu'il est mis en oeuvre en présence d'un catalyseur choisi parmi une amine tertiaire telle que la triéthylamine ou la tributylamine, (ou un sel organique de cette amine, tel que l'acétate de tributylamine), le dit catalyseur étant présent dans une proportion catalyseur/composé (I) allant de 0,05 à 1 , de préférence de 0,1 à 0,5.

## Patentansprüche

1. 2-Thiothiazolidin-5-on der allgemeinen Formel (I) 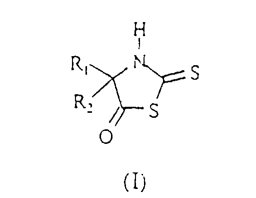 in der
- R¹ einen C₁-C₃-Alkyl- oder Phenylrest bedeutet,
- R² eine Gruppe bedeutet, die unter Phenyl oder Pyridyl ausgewählt ist und gegebenenfalls mit 1 bis 3 Gruppen substituiert ist, die unter Halogenatomen, Nitro-, Cyano-, C₁-C₃-Alkyl und C₁-C₃-Alkoxy ausgewählt sind;
ferner Salze sowie die entsprechenden Stereoisomeren und insbesondere, wenn die Reste R¹ und R² unterschiedlich sind, die optischen Isomeren, die sich aufgrund der Anwesenheit des asymmetrischen Kohlenstoffatoms, das R¹ und R² trägt, ergeben,
mit der Ausnahme von 4-Ethyl-4-phenyl-2-thio-thiazolidin-5-on.

2. Verbindung nach Anspruch 1, dadurch gekennzeichnet, daß
- R¹ einen C₁-C₃-Alkylrest bedeutet und
- R² einen gegebenenfalls durch ein Halogenatom oder durch eine Cyano-, Nitro-, Methyl- oder Methoxygruppe substituierten Phenylrest bedeutet.

3. Verbindung nach einem der Ansprüche 1 oder 2, dadurch gekennzeichnet, daß R² einen Phenylrest bedeutet und R¹ einen Methylrest bedeutet.

4. Verbindung nach einem der Ansprüche 1 bis 3, dadurch gekennzeichnet, daß es sich um ein in bezug auf das asymmetrische Kohlenstoffatom, das die Reste R¹ und R² trägt, Enantiomeres handelt.

5. Verfahren zur Herstellung der Verbindung der Formel (I) nach einem der Ansprüche 1 bis 4, dadurch gekennzeichnet, daß man eine Verbindung der Formel (II) mit Schwefelkohlenstoff in einem Lösungsmittel oder in einem Lösungsmittelgemisch, gegebenenfalls in Gegenwart einer Base, bei einer Temperatur von 0°C bis +50°C gemäß folgendem Reaktionsschema umsetzt: 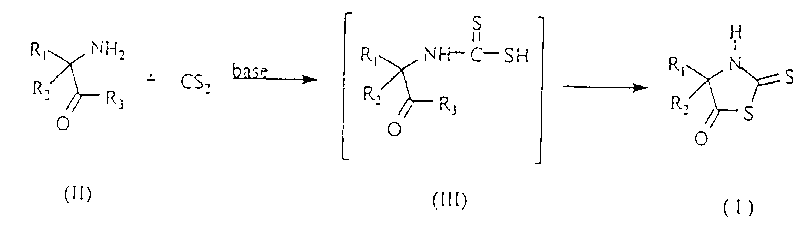 worin R³ Amino, Hydroxy oder lineares oder verzweigtes Alkoxy mit 1 bis 6 Kohlenstoffatomen und vorzugsweise mit 1 bis 3 Kohlenstoffatomen oder Benzyloxy, das gegebenenfalls durch ein Halogenatom substituiert ist, bedeutet.

6. Herstellungsverfahren nach Anspruch 5, dadurch gekennzeichnet, daß man das Verfahren in Abwesenheit einer Base oder bei einer Temperatur von 20 bis 40°C durchführt.

7. Verfahren zur Weiterverarbeitung der Verbindung der Formel (I) nach einem der Ansprüche 1 bis 4, dadurch gekennzeichnet, daß man eine Verbindung der Formel (V) in einem Lösungsmittel bei einer Temperatur von +20 bis +100°C und vorzugsweise von 40 bis 80°C gemäß folgendem Reaktionsschema umsetzt: 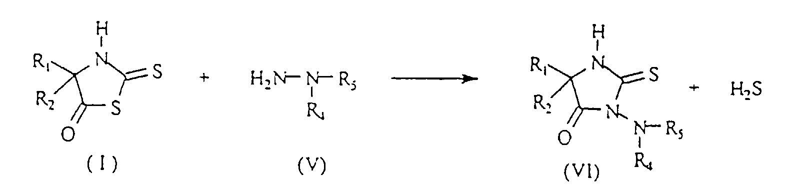 worin
- R⁴ ein Wasserstoffatom oder einen Acylrest bedeutet,
- R⁵ einen Aryl- oder Heteroarylrest bedeutet, der ausgewählt ist unter Phenyl, Naphthyl, Pyridinyl, Pyrimidinyl, Pyridazinyl, Pyrazinyl, Thienyl, Benzothienyl, Furyl, Benzofuryl, Chinolyl, Isochinolyl oder Methylendioxyphenyl, wobei jeder dieser Reste gegebenenfalls durch 1 bis 7 gleiche oder unterschiedliche Gruppen und vorzugsweise durch 1 bis 3 Gruppen substituiert ist, die unter den nachstehenden Bedeutungen von R⁵¹ ausgewählt sind;
- wobei R⁵¹ folgende Bedeutungen hat:
- ein Halogenatom oder
- ein Alkyl-, Halogenalkyl-, Alkoxy-, Halogenalkoxy-, Alkylthio-, Halogenalkylthio- oder Alkylsulfonylrest, die linear oder verzweigt sind und 1 bis 6 Kohlenstoffatome aufweisen, oder
- ein Cycloalkyl-, Halogencycloalkyl-, Alkenyloxy-, Alkinyloxy-, Alkinylthio- oder Alkinylthiorest mit 3 bis 6 Kohlenstoffatomen oder
- eine Nitro- oder Cyanogruppe oder
- ein Rest, der gegebenenfalls mit einem Alkyl- oder Acylrest mit 1 bis 6 Kohlenstoffatomen oder einem Alkoxycarbonylrest mit 2 bis 6 Kohlenstoffatomen mono- oder disubstituiert ist,

8. Weiterverarbeitungsverfahren nach Anspruch 7, dadurch gekennzeichnet, daß es in Gegenwart eines Katalysators durchgeführt wird, der unter tertiären Aminen, wie Triethylamin oder Tributylamin (oder einem organischen Salz dieser Amine, wie Tributylaminacetat) ausgewählt ist, wobei der Katalysator in einem Verhältnis Katalysator/Verbindung (I) von 0,05 bis 1 und vorzugsweise von 0,1 bis 0,5 vorhanden ist.

## Claims

1. 2-Thiothiazolidin-5-one compound of general formula (I): 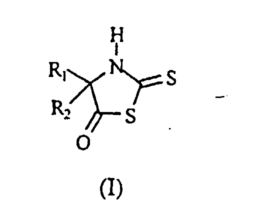 in which:
- R¹ is a C₁-C₃ alkyl or phenyl radical,
- R² is an aryl group chosen from phenyl or pyridyl, which is optionally substituted with 1 to 3 groups chosen from a halogen atom, a nitro or cyano group and a C₁-C₃ alkyl or C₁-C₃ alkoxy radical; as well as the salified forms and the corresponding stereoisomers and in particular, when the radicals R¹ and R² are different, the optical isomers resulting from the presence of the asymmetric carbon bearing R¹ and R²; with the exception of 4-ethyl-4-phenyl-2-thiothiazolidin-5-one.

2. Compound according to Claim 1, characterized in that :
- R¹ represents a C₁-C₃ alkyl radical,
- R² represents a phenyl optionally substituted with a halogen atom, a cyano or nitro group or a methyl or methoxy radical.

3. Compound according to either of Claims 1 and 2, characterized in that R² is a phenyl and R¹ is a methyl.

4. Compound according to one of Claims 1 to 3, characterized in that it is an enantiomer relative to the asymmetric carbon bearing R¹ and R².

5. Process for the preparation of the compound of formula (I) as defined in one of Claims 1 to 4, characterized in that a compound of formula (II) is reacted with carbon sulphide, in a solvent or a mixture of solvents, optionally in the presence of a base, at a temperature of between 0°C and + 50°C, according to the scheme: 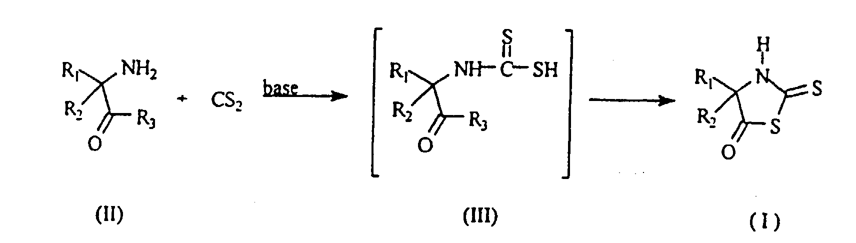 in which R³ is an amino or hydroxyl group or a linear or branched alkoxy radical containing from 1 to 6, preferably from 1 to 3, carbon atoms or a benzyloxy radical optionally substituted with a halogen atom.

6. Preparation process according to Claim 5, characterized in that it is carried out in the absence of base or at a temperature of between 20 and 40°C.

7. Process for the conversion of the compound of formula (I) as defined in one of Claims 1 to 4, characterized in that it is reacted with a compound of formula (V) in a solvent, and at a temperature of between + 20°C and + 100°C, preferably between 40 and 80°C, according to the scheme: 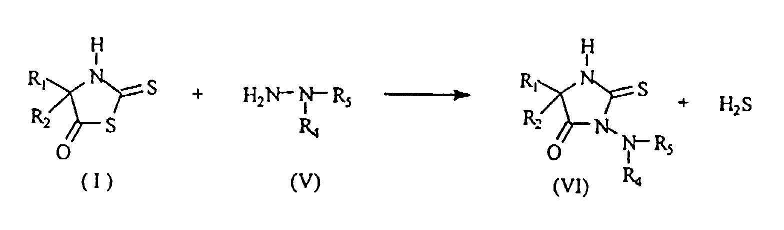 in which:
- R⁴ represents a hydrogen atom or an acyl radical;
- R⁵ represents an aryl or heteroaryl radical chosen from: phenyl, naphthyl, pyridyl, pyrimidinyl, pyridazinyl, pyrazinyl, thienyl, benzothienyl, furyl, benzofuryl, quinolyl, isoquinolyl or methylenedioxyphenyl, each of these radicals optionally being substituted with 1 to 7 groups, which may be identical or different, preferably from 1 to 3, chosen from the meanings of R⁵¹;
- R⁵¹ represents:
- a halogen atom or
- an alkyl, haloalkyl, alkoxy, haloalkoxy, alkylthio, haloalkylthio or alkylsulphonyl radical, which is linear or branched, of 1 to 6 carbon atoms, or
- a cycloalkyl, halocycloalkyl, alkenyloxy, alkynyloxy, alkenylthio or alkynylthio radical of 3 to 6 carbon atoms, or
- a nitro or cyano group, or
- an amino radical optionally mono- or disubstituted with an alkyl or acyl radical of 1 to 6 carbon atoms or alkoxycarbonyl radical of 2 to 6 carbon atoms.

8. Conversion process according to Claim 7, characterized in that it is carried out in the presence of a catalyst chosen from a tertiary amine such as triethylamine or tributylamine (or an organic salt of this amine, such as tributylamine acetate), the said catalyst being present in a catalyst/compound (I) proportion ranging from 0.05 to 1, preferably from 0.1 to 0.5.
